(19) Europäisches Patentamt
European Patent Office
Office européen des brevets

(11) Veröffentlichungsnummer: **0 066 774**
**B1**

(12) # EUROPÄISCHE PATENTSCHRIFT

(45) Veröffentlichungstag der Patentschrift: **12.12.84**

(21) Anmeldenummer: **82104549.9**

(22) Anmeldetag: **25.05.82**

(51) Int. Cl.³: **C 07 D 471/14,** A 61 K 31/55 // (C07D471/14, 243/00, 231/00, 221/00)

(54) **Neue 1,4,9,10-Tetrahydro-pyrazolo(4,3-e)pyrido(3,2-b)(1,4)-diazepin-10-one, Verfahren zu ihrer Herstellung und diese Verbindungen enthaltende Arzneimittel.**

(30) Priorität: **06.06.81 DE 3122670**

(43) Veröffentlichungstag der Anmeldung: **15.12.82 Patentblatt 82/50**

(45) Bekanntmachung des Hinweises auf die Patenterteilung: **12.12.84 Patentblatt 84/50**

(84) Benannte Vertragsstaaten: **AT BE CH DE FR IT LI LU NL SE**

(56) Entgegenhaltungen:
**EP - A - 0 023 707**
**DE - A - 1 645 956**
**DE - A - 2 707 269**

(73) Patentinhaber: **Dr. Karl Thomae GmbH, Postfach 1755, D-7950 Biberach (Riss) (DE)**

(72) Erfinder: **Schmidt, Günther, Dr. Dipl.-Chem., Johann-Sebastian-Bach-Strasse 27, D-7950 Biberach 1 (DE)**
Erfinder: **Engel, Wolfhard, Dr. Dipl.-Chem., Mozartstrasse 13, D-7950 Biberach 1 (DE)**
Erfinder: **Eberlein, Wolfgang, Dr. Dipl.-Chem., Obere Au 6, D-7950 Biberach 1 (DE)**
Erfinder: **Trummlitz, Günter, Dr. Dipl.-Chem., Buchenweg 27, D-7951 Warthausen (DE)**
Erfinder: **Engelhardt, Günther, Prof. Dr., Unterer Bühl 18, D-7950 Biberach 1 (DE)**

**EP 0 066 774 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue 1,4,9,10-Tetrahydro-pyrazolo[4,3-e]-pyrido[3,2-b][1,4]diazepin-10-one, Verfahren zu ihrer Herstellung und diese Verbindungen enthaltende Arzneimittel mit analgetischer, antiphlogistischer und antipyretischer Wirkung.

Die neuen Verbindungen besitzen die allgemeine Formel

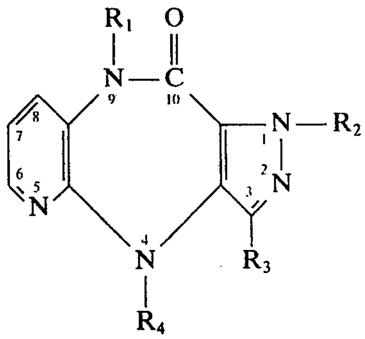

(I)

in der

$R_1$ ein Wasserstoffatom oder eine Alkylgruppe mit 1 bis 6 Kohlenstoffatomen,
$R_2$ eine Alkylgruppe mit 1 bis 3 Kohlenstoffatomen,
$R_3$ ein Wasserstoffatom oder eine Alkylgruppe mit 1 bis 3 Kohlenstoffatomen, und
$R_4$ ein Wasserstoffatom oder eine Alkylgruppe mit 1 bis 4 Kohlenstoffatomen bedeuten.

Bevorzugt sind solche Verbindungen der allgemeinen Formel I, in der
$R_1$ ein Wasserstoffatom, die Methyl-, Ethyl-, n-Propyl-, Isopropyl-, n-Butyl- oder Isobutylgruppe,
$R_2$ die Methylgruppe,
$R_3$ ein Wasserstoffatom oder die Methylgruppe und
$R_4$ ein Wasserstoffatom oder die Methylgruppe bedeuten.

Die Verbindungen der allgemeinen Formel I lassen sich wie folgt herstellen:

Durch Cyclisierung unter Halogenwasserstoffabspaltung eines N-(2-Halogen-3-pyridinyl)-4-amino-1H-pyrazol-5-carboxamids der allgemeinen Formel

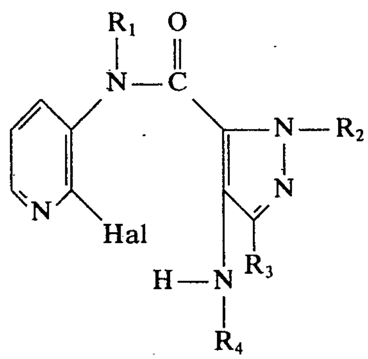

(II)

in der

$R_1$, $R_2$, $R_3$ und $R_4$ wie oben definiert sind und
Hal ein Halogenatom wie Chlor, Brom oder Iod, vorzugsweise ein Chloratom, darstellt.

Die Cyclisierung erfolgt in einem organischen Lösungsmittel bei Temperaturen zwischen 80 und 200°C, vorzugsweise zwischen 100 und 150°C. Als Lösungsmittel sind besonders höher siedende Lösungsmittel, wie Sulfolan oder 1,2,4-Trichlorbenzol, geeignet. Die Zugabe katalytischer Mengen einer Mineralsäure, wie Schwefelsäure, Chlorwasserstoff- oder Phosphorsäure, ist für die Cyclisierungsreaktion von Vorteil.

Erhält man erfindungsgemäß eine Verbindung der allgemeinen Formel I, in der $R_4$ wie eingangs definiert ist und $R_1$ ein Wasserstoffatom darstellt, so kann diese mittels Alkylierung in eine entsprechende Verbindung der allgemeinen Formel I, in der $R_4$ wie eingangs definiert ist und $R_1$ eine Alkylgruppe mit 1 bis 6 Kohlenstoffatomen darstellt, übergeführt werden
oder eine Verbindung der allgemeinen Formel I, in der $R_1$ eine Alkylgruppe mit 1 bis 6 Kohlenstoffatomen und $R_4$ ein Wasserstoffatom darstellen, so kann diese mittels Alkylierung in eine entsprechende Verbindung der allgemeinen Formel I, in der $R_1$ eine Alkylgruppe mit 1 bis 6 Kohlenstoffatomen und $R_4$ eine Alkylgruppe mit 1 bis 4 Kohlenstoffatomen darstellen, übergeführt werden
oder eine Verbindung der allgemeinen Formel I, in der $R_1$ und $R_4$ je ein Wasserstoffatom darstellen, so kann diese mittels Alkylierung in eine entsprechende Verbindung der allgemeinen Formel I, in der $R_1$ und $R_4$ jeweils eine Alkylgruppe mit 1 bis 4 Kohlenstoffatomen darstellen, übergeführt werden.

Die Alkylierung erfolgt beispielsweise unter Verwendung von Dialkylsulfaten oder Alkylhalogeniden, vorzugsweise von Alkyliodiden, in Gegenwart von Metallisierungsmitteln, wie Natriumamid oder

Natriumhydrid, und eines geeigneten Lösungsmittels. Die Ausgangsverbindungen der allgemeinen Formel II sind auf folgendem Weg zugänglich:

Ein 3-Amino-2-halogen-pyridin der allgemeinen Formel

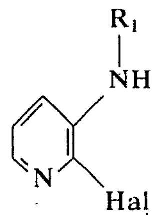

(III)

in der

$R_1$ und Hal die obigen Bedeutungen besitzen, wird mit einer 4-Nitro-1 H-pyrazol-5-carbonsäure der allgemeinen Formel

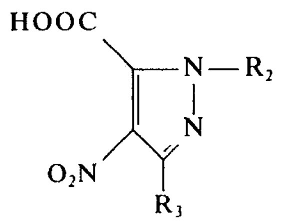

(IV)

in der

$R_2$ und $R_3$ wie eingangs erwähnt definiert sind, umgesetzt

entweder

mittelt Thionylchlorid in einem geeigneten Lösungsmittel wie Dioxan oder Hexamethylphosphorsäuretriamid bei Temperaturen zwischen 60 und 90°C

oder

mittels Phosphoroxidchlorid und einer organischen Base wie Triethylamin in einem geeigneten Lösungsmittel, wie Toluol oder Dioxan, bei Temperaturen zwischen 80 und 130°C.

Das dabei erhaltene N-(2-Halogen-3-pyridinyl)-4-nitro-1 H-pyrazol-5-carboxamid der allgemeinen Formel

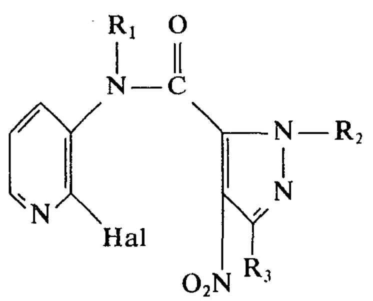

(V)

wird anschließend zu der Ausgangsverbindung der allgemeinen Formel II reduziert. Die Reduktion der Nitro- zu der Aminogruppe erfolgt beispielsweise dadurch, daß eine Verbindung der allgemeinen Formel V in einem geeigneten Lösungsmittel, wie beispielsweise Dioxan, Methanol oder Ethanol, in Gegenwart der üblichen Hydrierkatalysatoren, bevorzugt Raney-Nickel, und bei Temperaturen zwischen 0 und 100°C und einem Wasserstoffdruck zwischen 1 und 200 bar hydriert wird.

Die Verbindungen der allgemeinen Formel I besitzen sehr gute pharmakologische Wirkungen, insbesondere eine sehr gute analgetische, antiphlogistische und antipyretische Wirkung; einige Verbindungen zeigen darüber hinaus eine positiv inotrope, diuretische und/oder spasmolytische, sedative, muskelrelaxierende und anxiolytische Wirkung. Außerdem stellen die Verbindungen der allgemeinen Formel I, in der $R_1$ bis $R_3$ wie eingangs definiert sind und $R_4$ ein Wasserstoffatom darstellt, wertvolle Zwischenprodukte dar, z. B. für die Herstellung der Verbindungen der vorliegenden allgemeinen Formel I, in der $R_1$ eine Alkylgruppe mit 1 bis 6 Kohlenstoffatomen und/oder $R_4$ eine Alkylgruppe mit 1 bis 4 Kohlenstoffatomen darstellen.

Beispielsweise wurden folgende Verbindungen

1,3-Dimethyl-1,4,9,10-tetrahydro-pyrazolo[4,3-e]pyrido[3,2-b][1,4]diazepin-10-on = A
1,4,9,10-Tetrahydro-1,3,9-trimethyl-pyrazolo[4,3-e]pyrido[3,2-b][1,4]diazepin-10-on = B
1,3-Dimethyl-9-ethyl-1,4,9,10-tetrahydro-pyrazolo[4,3-e]pyrido[3,2-b][1,4]diazepin-10-on = C
1,4,9,10-Tetrahydro-1,3,4,9-tetramethyl-pyrazolo[4,3-e]pyrido[3,2-b][1,4]diazepin-10-on = D
9-Ethyl-1,4,9,10-tetrahydro-1,3,4-trimethyl-pyrazolo[4,3-e]pyrido[3,2-b][1,4]diazepin-10-on = E

und

9-(n-Butyl)-1,3-dimethyl-1,4,9,10-tetrahydro-pyrazolo[4,3-e]pyrido[3,2-b][1,4]diazepin-10-on = F

auf ihre analgetische Wirkung gegen den Entzündungsschmerz der Ratte, gegen den Wärmeschmerz der Maus und auf eine akute Toxizität an der Maus untersucht.

## Methodik

### 1. Wirkung gegen den Entzündungsschmerz der Ratte

Die Prüfung erfolgte in der Versuchsanordnung nach Randall-Selitto (Arch. int. Pharmacodyn. 111, 409 (1957)) an männlichen Ratten mit einem Gewicht zwischen 100 und 130 g. Der Entzündungsschmerz wurde durch die subplantare Injektion von 0,1 ml einer Suspension von 1,12 g Trockenhefe in 18,9 ml einer 5,5%igen Glukoselösung in eine Hinterpfote ausgelöst.

Die Prüfsubstanzen wurden als Verreibung in 1%iger Methylzellulose 90 min nach der Gabe des Phlogisticums, d. h. 90 min vor Bestimmung der Schmerzschwelle, per Schlundsonde beigebracht.

Aus der nach den verschiedenen Dosen der Prüfsubstanz gefundenen Schmerzschwelle wurde nach linearer Regressionsanalyse nach Linder (Statistische Methoden, 4. Auflage, pp. 148–162, Birkhäuser Basel 1964) eine $ED_{50}$ als jene Dosis berechnet, die eine Anhebung der Schmerzschwelle um 50% bewirkte.

### 2. Wirkung gegen den Wärmeschmerz der Maus

Es wurde eine Variante der Methode von Chen und Beckman (Science 113, 631 (1951)) angewendet. Die Oberflächentemperatur der heißen Platte betrug 52°C.

Männliche Mäuse erhielten die Prüfsubstanz als Verreibung in 1%iger Methylcellulose per Schlundsonde.

Aus der durch die verschiedenen Dosen der Prüfsubstanzen bewirkten Verlängerung der Zeit bis zum Einsetzen von Abwehrreaktionen (Reaktionszeit) wurde nach linearer Regressionsanalyse nach Linder (siehe oben) eine $ED_{100}$ als jene Dosis berechnet, die zu einer Verdoppelung der primären Reaktionszeit vor Behandlung führte.

### 3. Akute Toxizität an der Maus

Mäuse beider Geschlechter (1 : 1) erhielten die Prüfsubstanzen als Verreibung in 1%iger Methylcellulose (0,2 ml/10 g) per Schlundsonde beigebracht.

Aus dem Prozentsatz der Tiere, die nach den verschiedenen Dosen innerhalb von 14 Tagen verstarben, wurde soweit möglich nach Litchfield und Wilcoxon (J. Pharmacol. exp. Ther. 96, 99 (1949)) eine $LD_{50}$ berechnet.

## Ergebnisse

Die bei diesen Prüfungen erhobenen Befunde sind in den nachfolgenden Tabellen zusammengestellt. Die geprüften Verbindungen zeigen an Maus und Ratte in einem untoxischen Dosenbereich sehr gute analgetische Wirkungsqualitäten.

Tabelle 1

Wirkung gegen den Entzündungsschmerz der Ratte in der Versuchsanordnung nach Randall-Selitto 90 Minuten nach oraler Gabe:

| Substanz | $ED_{50}$ [mg/kg] |
|---|---|
| A | 26 |
| B | 11 |
| C | 28 |
| D | 7,4 |
| E | 5,9 |
| F | 139 |

Tabelle 2

Wirkung gegen den Wärmeschmerz der Maus (hot-plate-Technik) 30 bis 120 Minuten nach oraler Gabe:

| Substanz | $ED_{100}$ [mg/kg] |
|---|---|
| A | 15 |
| B | 30 |
| C | 28 |
| F | 87 |

Tabelle 3

Akute Toxizität an der Maus nach oraler Gabe.

| Substanz | $LD_{50}$ [mg/kg] |
|---|---|
| A | 152 |
| B | 175 |
| C | 171 |
| D | 225 |
| E | 202 |
| F | >1000*) |

*) Nach der Dosis von 1000 mg/kg 1 von 8 Tieren verstorben.

Aufgrund ihrer pharmakologischen Eigenschaften eignen sich die erfindungsgemäß hergestellten Verbindungen zur Verwendung als Arzneimittel mit analgetischen, antiphlogistischen und antipyretischen Wirkungen. Hierzu lassen sich die neuen Verbindungen, gegebenenfalls in Kombination mit anderen Wirksubstanzen wie N-Butyl-scopolaminium-bromid, Codeinphosphat, Amobarbital, Acetylsalicylsäure und/oder Coffein, in die üblichen galenischen Zubereitungsformen wie Tabletten, Dragées oder Suppositorien einarbeiten. Die Dosis pro Arzneimitteleinheit beträgt dabei 10 bis 200 mg, vorzugsweise 50 bis 100 mg, und die Tagesdosis 30 bis 600 mg, vorzugsweise 150 bis 300 mg Wirksubstanz.

Die nachfolgenden Beispiele sollen die Erfindung näher erläutern:

## Herstellung der Ausgangsprodukte

### Beispiel A

N-(2-Chlor-3-pyridinyl)-1,3-dimethyl-4-nitro-1 H-pyrazol-5-carboxamid

a) Zu einer Suspension von 1,85 g (0,01 Mol) 1,3-Dimethyl-4-nitro-1 H-pyrazol-5-carbonsäure in 2 ml Hexamethylphosphorsäuretriamid wurden bei Raumtemperatur 0,72 ml Thionylchlorid zugetropft und 20 Minuten bei 60°C gerührt. Zur entstandenen Lösung tropfte man eine Lösung von 1,28 g (0,01 Mol) 2-Chlor-3-aminopyridin in 2 ml Hexamethylphosphorsäuretriamid zu. Man erhitzte 45 Minuten auf 70°C und goß dann auf Eis. Die abgeschiedenen weißen Kristalle wurden abgesaugt, mit Wasser gewaschen, mit verdünnter Natriumhydrogencarbonat-Lösung verrührt und abgesaugt. Nach dem Trocknen erhielt man 2,3 g (78% der Theorie) des gewünschten Säureamids vom Schmelzpunkt 183 bis 185°C.

b) Die gleiche Substanz wurde auch auf folgende Weise erhalten:

Zu einer Lösung von 1,85 g (0,01 Mol) 1,3-Dimethyl-4-nitro-1 H-pyrazol-5-carbonsäure, 1,28 g (0,01 Mol) 2-Chlor-3-aminopyridin und 1,7 ml (0,01 Mol) Triethylamin in 25 ml Toluol wurde unter Rückfluß innerhalb von 60 Minuten eine Lösung von 0,52 ml Phosphoroxidchlorid in 5 ml Toluol zugetropft. Man rührte 90 Minuten bei 100°C, kühlte ab und setzte 50 ml Wasser zu. Der kristalline Niederschlag wurde abgesaugt, mit verdünnter Natriumhydrogencarbonat-Lösung verrührt, abgesaugt und mit Wasser gewaschen. Man erhielt 1,8 g (60% der Theorie) des gewünschten Säureamids vom Schmelzpunkt 183 bis 185°C.

### Beispiel B

4-Amino-N-(2-chlor-3-pyridinyl)-1,3-dimethyl-1 H-pyrazol-5-carboxamid

2,0 g (0,0068 Mol) N-(2-Chlor-3-pyridinyl)-1,3-dimethyl-4-nitro-1 H-pyrazol-5-carboxamid wurden in 30 ml Dioxan mit 0,7 g Raney-Nickel als Katalysator bei 70°C und 30 bar hydriert. Nach beendeter Wasserstoffaufnahme wurde der Katalysator abfiltriert und im Vakuum das Dioxan abdestilliert. Der kristalline Rückstand wurde aus n-Propanol umkristallisiert. Man erhielt 1,3 g (82% der Theorie) der gewünschten Aminoverbindung vom Schmelzpunkt 196 bis 198°C.

### Beispiel C

N-(2-Chlor-3-pyridinyl)-4-(methylamino)-N,1,3-trimethyl-1 H-pyrazol-5-carboxamid

Die Mischung aus 40,8 g (0,091 Mol) N-(2-Chlor-3-pyridinyl)-4-[[(4-methylphenyl)sulfonyl]methylamino]-N,1,3-trimethyl-1 H-pyrazol-5-carboxamid (hergestellt aus N-(2-Chlor-3-pyridinyl)-4-[[(4-methylphenyl)sulfonyl]amino]-N,1,3-trimethyl-1 H-pyrazol-5-carboxamid durch Umsetzung mit Natriumhydrid in wasserfreiem Dimethylformamid und anschließende Einwirkung von Methyliodid) und 0,6 kg Polyphosphorsäure (Gehalt an Phosphorpentoxid: 85%) wurde unter Rühren 6 Stunden auf 60°C erwärmt. Die noch warme Reaktionsmischung wurde in 1,6 kg gestoßenes Eis eingerührt und anschließend unter äußerer Kühlung mit Eiswasser mit 40% wässeriger Natriumhydroxid-Lösung bis zur

Beendigung der Fällungsreaktion versetzt. Der noch stark saure Ansatz wurde nach 2stündigem Stehenlassen bei +5°C abgenutscht. Der Filterrückstand wurde sorgfältig mit Wasser gewaschen, anschließend an der Luft getrocknet.
Ausbeute: 20,9 g (78% der Theorie).

Beispiel D

4-Amino-N-(2-chlor-3-pyridinyl)-1-methyl-1 H-pyrazol-5-carboxamid

a) 1-Methyl-4-nitro-1 H-pyrazol-5-carbonsäure

In eine Mischung von 200 ml konz. Schwefelsäure und 60 ml 90% rauchender Salpetersäure wurden unter Rühren 69,0 g (0,547 Mol) 1-Methyl-1 H-pyrazol-5-carbonsäure vom Schmelzpunkt 227 bis 228°C (R. Hüttel und M. E. Schön, Liebigs Ann. Chem. 625, 55 [1959]) in kleinen Portionen eingetragen. Die Temperatur der Mischung stieg dabei von anfangs 75°C auf maximal 95°C. Nach Abklingen der exothermen Reaktion wurde noch 30 Minuten auf 95°C erwärmt. Das erkaltete Nitriergemisch wurde in 300 g gestoßenes Eis eingerührt, die erhaltene Suspension noch eiskalt abgenutscht. Der Filterrückstand wurde mit Wasser gewaschen und ergab nach dem Umkristallisieren aus Ethylacetat 44,0 g (47% der Theorie) an fahlgelben Kristallen vom Schmelzpunkt 169 bis 170°C (Zers.).

b) N-(2-Chlor-3-pyridinyl)-1-methyl-4-nitro-1 H-pyrazol-5-carboxamid

Hergestellt analog Beispiel Aa aus 1-Methyl-4-nitro-1 H-pyrazol-5-carbonsäure, Thionylchlorid und 2-Chlor-3-aminopyridin in Hexamethylphosphorsäuretriamid.
Ausbeute: 86% der Theorie,
Schmelzpunkt: 150 bis 151°C.

c) 4-Amino-N-(2-chlor-3-pyridinyl)-1-methyl-1 H-pyrazol-5-carboxamid

Die Lösung von 62,0 g (0,22 Mol) N-(2 Chlor-3-pyridinyl)-1-methyl-4-nitro-1 H-pyrazol-5-carboxamid in 1 l Dioxan wurde in Gegenwart von 5 g 10% Palladium/Tierkohle als Katalysator bei Zimmertemperatur und 5 bar bis zur Beendigung der Wasserstoffaufnahme hydriert. Man filtrierte vom Katalysator und dem entstandenen Niederschlag ab und dampfte das Filtrat im Vakuum ein. Der verbleibende Destillationsrückstand wurde aus Methanol umkristallisiert. Man erhielt 25,0 g (45% der Theorie) an farblosen Kristallen vom Schmelzpunkt 124 bis 125°C.

Herstellung der Endprodukte

Beispiel 1

1,3-Dimethyl-1,4,9,10-tetrahydro-pyrazolo[4,3-e]pyrido[3,2-b][1,4]diazepin-10-on

25,7 g (0,0966 Mol) 4-Amino-N-(2-chlor-3-pyridinyl)-1,3-dimethyl-1 H-pyrazol-5-carboxamid und 0,5 ml konzentrierte Schwefelsäure wurden in 50 ml Sulfolan 2,5 Stunden bei 130°C gerührt. Nach dem Abkühlen wurde der Kristallbrei abgesaugt und mit n-Propanol gewaschen. Dann verrührte man mit verdünntem wässerigem Ammoniak und saugte ab. Man erhielt 21,3 g (96% der Theorie) der gewünschten Verbindung vom Schmelzpunkt 197 bis 199°C (aus Ethylacetat).

Beispiel 2

1,4,9,10-Tetrahydro-1,3,9-trimethyl-pyrazolo[4,3-e]pyrido[3,2-b][1,4]diazepin-10-on

5,25 g (0,025 Mol) 1,3-Dimethyl-1,4,9,10-tetrahydro-pyrazolo[4,3-e]pyrido[3,2-b][1,4]diazepin-10-on wurden in 25 ml Dimethylformamid gelöst und mit 1,2 g einer 55%igen Natriumhydrid-Dispersion in Mineralöl versetzt und 2 Stunden bei 60°C gerührt. Nach dem Abkühlen auf 40°C wurden 2,2 ml Methyliodid zugetropft, die Mischung anschließend 3 Stunden bei 40°C gerührt. Nach der Zugabe von 50 ml Wasser und Kühlen im Eisbad kristallisierte die gewünschte Verbindung aus. Man saugte ab und wusch mit Petrolether das Mineralöl heraus. Man erhielt 3,0 g (50% der Theorie) der gewünschten Verbindung vom Schmelzpunkt 141 bis 141,5°C (aus wässerigem Ethanol).

Beispiel 3

1,3-Dimethyl-9-ethyl-1,4,9,10-tetrahydro-pyrazolo[4,3-e]pyrido[3,2-b][1,4]diazepin-10-on

Die Herstellung erfolgt gemäß Beispiel 2 aus 1,3-Dimethyl-1,4,9,10-tetrahydro-pyrazolo[4,3-e]-pyrido[3,2-b][1,4]diazepin-10-on, Natriumhydrid und Ethyliodid in Dimethylformamid. Das Rohprodukt wurde säulenchromatographisch gereinigt (Kieselgel; Fließmittel: Methylenchlorid+Ethylacetat=4+1).
Schmelzpunkt: 137 bis 138°C (aus wässerigem Ethanol),
Ausbeute: 62% der Theorie.

Beispiel 4

1,4,9,10-Tetrahydro-1,3,4,9-tetramethyl-pyrazolo[4,3-e]pyrido[3,2-b][1,4]diazepin-10-on

4,6 g (0,02 Mol) 1,3-Dimethyl-1,4,9,10-tetrahydro-pyrazolo[4,3-e]pyrido[3,2-b][1,4]diazepin-10-on wurden in 20 ml Dimethylformamid gelöst und mit 2,2 g einer 55%igen Natriumhydrid-Dispersion in Mineralöl versetzt und 30 Minuten bei 60°C gerührt. Nach dem Abkühlen auf 40°C wurden 3,7 ml Methyliodid zugetropft und weitere 2 Stunden bei 40°C gerührt. Der entstandene Kristallbrei wurde mit 50 ml Wasser versetzt, im Eisbad auf 0°C abgekühlt und abgesaugt. Nach der Umkristallisation aus wässerigem Ethanol erhielt man 3 g der gewünschten Verbindung vom Schmelzpunkt 147 bis 148,5°C.
Ausbeute: 59% der Theorie.

Beispiel 5

1,4,9,10-Tetrahydro-1,3,4,9-tetramethyl-pyrazolo[4,3-e]pyrido[3,2-b][1,4]diazepin-10-on

5,9 g N-(2-Chlor-3-pyridinyl)-N-methyl-1,3-dimethyl-4-methylamino-1H-pyrazol-5-carboxamid wurden in 6 ml 1,2,4-Trichlorbenzol 5 Stunden lang bei 180°C gerührt. Nach dem Abkühlen wurde der Kristallbrei abgesaugt, mit Cyclohexan gewaschen und aus wässerigem Ethanol umkristallisiert.
Schmelzpunkt: 147 bis 148,5°C,
Ausbeute: 74% der Theorie.

Beispiel 6

9-Ethyl-1,4,9,10-tetrahydro-1,3,4-trimethyl-pyrazolo[4,3-e]pyrido[3,2-b][1,4]diazepin-10-on

6,0 g 1,3-Dimethyl-9-ethyl-1,4,9,10-tetrahydro-pyrazolo[4,3-e]pyrido[3,2-b][1,4]diazepin-10-on wurden in 30 ml Dimethylformamid gelöst und mit 1,1 g einer 55%igen Natriumhydrid-Dispersion in Mineralöl versetzt und 2 Stunden bei 60°C gerührt. Nach dem Abkühlen auf 40°C wurden 1,9 ml Methyliodid zugetropft und anschließend 2 Stunden bei 40°C gerührt. Das Reaktionsgemisch wurde im Vakuum eingedampft. Der Rückstand wurde säulenchromatographisch gereinigt (Kieselgel; Fließmittel: Methylenchlorid+Ethylacetat = 11+5).
Schmelzpunkt: 127 bis 129°C (aus wässerigem Ethanol),
Ausbeute: 75% der Theorie.

Beispiel 7

9-(n-Butyl)-1,3-dimethyl-1,4,9,10-tetrahydro-pyrazolo[4,3-e]pyrido[3,2-b][1,4]diazepin-10-on

Die Herstellung erfolgte gemäß Beispiel 3 aus 1,3-Dimethyl-1,4,9,10-tetrahydro-pyrazolo[4,3-e]-pyrido[3,2-b][1,4]diazepin-10-on, Natriumhydrid und n-Butylbromid in Dimethylformamid mit nachfolgender chromatographischer Reinigung.
Schmelzpunkt: 144 bis 146°C (aus Ethylacetat),
Ausbeute: 67% der Theorie.

Beispiel 8

9-(n-Butyl)-1,4,9,10-tetrahydro-1,3,4-trimethyl-pyrazolo[4,3-e]pyrido[3,2-b][1,4]diazepin-10-on

Die Herstellung erfolgte gemäß Beispiel 6 aus 9-(n-Butyl)-1,3-dimethyl-1,4,9,10-tetrahydro-pyrazolo[4,3-e]pyrido[3,2-b][1,4]diazepin-10-on, Natriumhydrid und Methyliodid in Dimethylformamid mit nachfolgender chromatographischer Reinigung.
Schmelzpunkt: 93 bis 93,5°C (aus Petrolether),
Ausbeute: 61% der Theorie.

Beispiel 9

1-Methyl-1,4,9,10-tetrahydro-pyrazolo[4,3-e]pyrido[3,2-b][1,4]diazepin-10-on

25,0 g (0,099 Mol) 4-Amino-N-(2 chlor-3-pyridinyl)-1-methyl-1H-pyrazol-5-carboxamid wurden in 100 ml Sulfolan gelöst und nach Zugabe von 5 Tropfen konz. Schwefelsäure 1 Stunde auf 120°C erhitzt. Die erkaltete Reaktionsmischung wurde filtriert, der Filterrückstand in 5% wässeriger Ammoniak-Lösung suspendiert und abermals filtriert. Das erhaltene Produkt wurde gründlich mit Wasser gewaschen, dann mit Methanol verrührt und kalt abgenutscht. Nach dem Trocknen schmolzen die farblosen Kristalle bei 304 bis 305°C.
Ausbeute: 14,6 g (68,5% der Theorie).

Beispiel 10

1,3-Dimethyl-9-(n-propyl)-1,4,9,10-tetrahydro-pyrazolo[4,3-e]pyrido[3,2-b][1,4]diazepin-10-on

Die Herstellung erfolgte gemäß Beispiel 2 aus 1,3-Dimethyl-1,4,9,10-tetrahydro-pyrazolo[4,3-e]-pyrido[3,2-b][1,4]diazepin-10-on, Natriumhydrid und n-Propylbromid in Dimethylformamid mit nachfolgender chromatographischer Reinigung (Kieselgel; Fließmittel: Methylenchlorid+Ethylacetat=4+1).
Schmelzpunkt: 196 bis 197,5°C (aus Ethylacetat),
Ausbeute: 61% der Theorie.

Beispiel 11

1,3-Dimethyl-9-isopropyl-1,4,9,10-tetrahydro-pyrazolo[4,3-e]pyrido[3,2-b][1,4]diazepin-10-on

Die Herstellung erfolgte gemäß Beispiel 2 aus 1,3-Dimethyl-1,4,9,10-tetrahydro-pyrazolo[4,3-e]-pyrido[3,2-b][1,4]diazepin-10-on, Natriumhydrid und Isopropylbromid in Dimethylformamid mit nachfolgender chromatographischer Reinigung (Kieselgel; Fließmittel: Methylenchlorid+Ethylacetat=4+1).
Schmelzpunkt: 155 bis 157°C (aus Ethylacetat),
Ausbeute: 56% der Theorie.

Beispiel 12

1,3-Dimethyl-9-isobutyl-1,4,9,10-tetrahydro-pyrazolo[4,3-e]pyrido[3,2-b][1,4]diazepin-10-on

Die Herstellung erfolgte gemäß Beispiel 2 aus 1,3-Dimethyl-1,4,9,10-tetrahydro-pyrazolo[4,3-e]-pyrido[3,2-b][1,4]diazepin-10-on, Natriumhydrid und Isobutylbromid in Dimethylformamid mit nachfolgender chromatographischer Reinigung (Kieselgel; Fließmittel: Methylenchlorid+Ethylacetat=4+1).
Schmelzpunkt: 180 bis 182°C (aus Ethylacetat),
Ausbeute: 63% der Theorie.

Beispiel I

Tabletten mit 50 mg 1,4,9,10-Tetrahydro-1,3,9-trimethyl-pyrazolo[4,3-e]-pyrido[3,2-b][1,4]diazepin-10-on

Zusammensetzung:
1 Tablette enthält:

| | |
|---|---|
| Wirkstoff | 50,0 mg |
| Milchzucker | 128,0 mg |
| Kartoffelstärke | 40,0 mg |
| Magnesiumstearat | 2,0 mg |
| | 220,0 mg |

Herstellungsverfahren

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 50 mg 1,4,9,10-Tetrahydro-1,3,9-trimethyl-pyrazolo[4,3-e]-pyrido[3,2-b][1,4]diazepin-10-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg.

Beispiel III

Suppositorien mit 70 mg 1,4,9,10-Tetrahydro-1,3,9-trimethyl-pyrazolo[4,3-e]-pyrido[3,2-b][1,4]diazepin-10-on

Zusammensetzung:
1 Zäpfchen enthält:

| | |
|---|---|
| Wirkstoff | 70,0 mg |
| Zäpfchenmasse (z. B. Witepol W 45®) | 1630,0 mg |
| | 1700,0 mg |

Herstellungsverfahren

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus.
Zäpfchengewicht: 1,7 g.

**Patentansprüche für die Vertragsstaaten: BE, CH, DE, FR, IT, LI, LU, NL, SE**

1. 1,4,9,10-Tetrahydro-pyrazolo[3,4-e]pyrido[3,2-b][1,4]diazepin-10-one der allgemeinen Formel

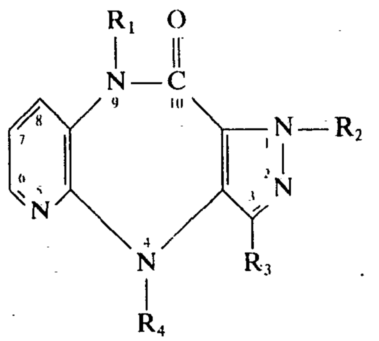

(I)

in der
$R_1$ ein Wasserstoffatom oder eine Alkylgruppe mit 1 bis 6 Kohlenstoffatomen,
$R_2$ eine Alkylgruppe mit 1 bis 3 Kohlenstoffatomen,
$R_3$ ein Wasserstoffatom oder eine Alkylgruppe mit 1 bis 3 Kohlenstoffatomen, und
$R_4$ ein Wasserstoffatom oder eine Alkylgruppe mit 1 bis 4 Kohlenstoffatomen bedeuten.

2. Verbindungen der allgemeinen Formel I gemäß Anspruch 1, dadurch gekennzeichnet, daß
$R_1$ ein Wasserstoffatom oder eine Alkylgruppe mit 1 bis 3 Kohlenstoffatomen,
$R_2$ eine Alkylgruppe mit 1 bis 3 Kohlenstoffatomen,
$R_3$ ein Wasserstoffatom oder eine Alkylgruppe mit 1 bis 3 Kohlenstoffatomen, und
$R_4$ ein Wasserstoffatom bedeuten.

3. Verbindungen der allgemeinen Formel I gemäß Anspruch 1, dadurch gekennzeichnet, daß
$R_1$ ein Wasserstoffatom, die Methyl-, Ethyl-, n-Propyl-, Isopropyl-, n-Butyl- oder Isobutylgruppe,
$R_2$ die Methylgruppe,
$R_3$ ein Wasserstoffatom oder die Methylgruppe und
$R_4$ ein Wasserstoffatom oder die Methylgruppe bedeuten.

4. Verfahren zur Herstelung von 1,4,9,10-Tetrahydro-pyrazolo[4,3-e]pyrido[3,2-b][1,4]diazepin-10-onen der allgemeinen Formel

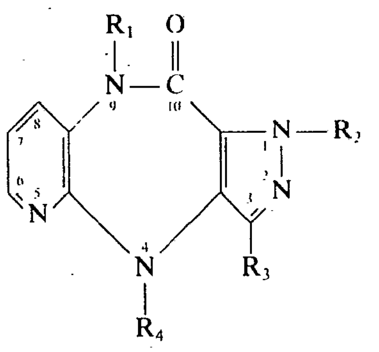

(I)

in der
$R_1$ ein Wasserstoffatom oder eine Alkylgruppe mit 1 bis 6 Kohlenstoffatomen,
$R_2$ eine Alkylgruppe mit 1 bis 3 Kohlenstoffatomen,
$R_3$ ein Wasserstoffatom oder eine Alkylgruppe mit 1 bis 3 Kohlenstoffatomen, und
$R_4$ ein Wasserstoffatom oder eine Alkylgruppe mit 1 bis 4 Kohlenstoffatomen bedeuten, dadurch gekennzeichnet, daß ein N-(2-Halogen-3-pyridinyl)-4-amino-1H-pyrazol-5-carboxamid der allgemeinen Formel

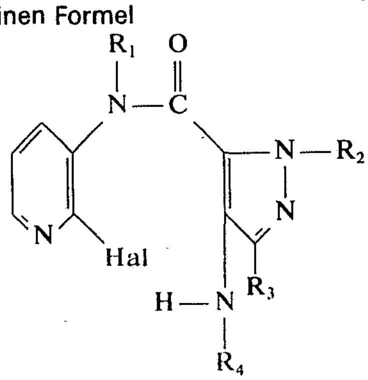

(II)

in der $R_1$, $R_2$, $R_3$ und $R_4$ wie oben definiert sind und Hal ein Halogenatom darstellt, in einem organischen Lösungsmittel bei Temperaturen zwischen 80 und 200°C, gegebenenfalls in Gegenwart katalytischer Mengen einer Mineralsäure, cyclisiert wird
und gewünschtenfalls anschließend eine so erhaltene Verbindung der allgemeinen Formel I, in der $R_4$ wie eingangs definiert ist und $R_1$ ein Wasserstoffatom darstellt, mittels Alkylierung in eine entsprechende Verbindung der allgemeinen Formel I, in der $R_4$ wie eingangs definiert ist und $R_1$ eine Alkylgruppe mit 1 bis 6 Kohlenstoffatomen darstellt, übergeführt wird
oder eine Verbindung der allgemeinen Formel I, in der $R_1$ eine Alkylgruppe mit 1 bis 6 Kohlenstoffatomen und $R_4$ ein Wasserstoffatom darstellen, mittels Alkylierung in eine entsprechende Verbindung der allgemeinen Formel I, in der $R_1$ eine Alkylgruppe mit 1 bis 6 Kohlenstoffatomen und $R_4$ eine Alkylgruppe mit 1 bis 4 Kohlenstoffatomen darstellen, übergeführt wird
oder eine Verbindung der allgemeinen Formel I, in der $R_1$ und $R_4$ je ein Wasserstoffatom darstellen, mittels Alkylierung in eine entsprechende Verbindung der allgemeinen Formel I, in der $R_1$ und $R_4$ jeweils eine Alkylgruppe mit 1 bis 4 Kohlenstoffatomen darstellen, übergeführt wird.

5. Arzneimittel, enthaltend eine oder mehrere Verbindungen der allgemeinen Formel I gemäß den Ansprüchen 1 bis 3 neben üblichen Träger- und/oder Hilfsstoffen.

**Patentansprüche für den Vertragsstaat: AT**

1. Verfahren zur Herstellung von 1,4,9,10-Tetrahydro-pyrazolo[3,4-e]pyrido[3,2-b][1,4]diazepin-10-onen der allgemeinen Formel

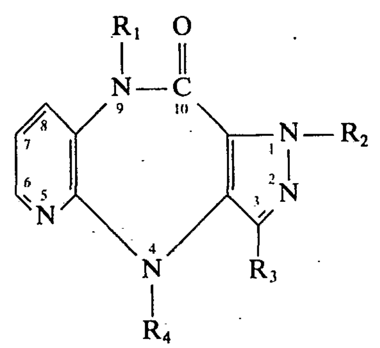

(I)

in der
$R_1$ ein Wasserstoffatom oder eine Alkylgruppe mit 1 bis 6 Kohlenstoffatomen,
$R_2$ eine Alkylgruppe mit 1 bis 3 Kohlenstoffatomen,
$R_3$ ein Wasserstoffatom oder eine Alkylgruppe mit 1 bis 3 Kohlenstoffatomen, und
$R_4$ ein Wasserstoffatom oder eine Alkylgruppe mit 1 bis 4 Kohlenstoffatomen bedeuten, dadurch gekennzeichnet, daß ein N-(2-Halogen-3-pyridinyl)-4-amino-1H-pyrazol-5-carboxamid der allgemeinen Formel

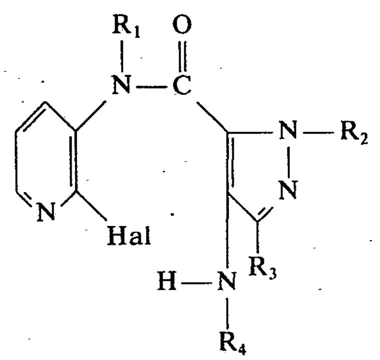

(II)

in der
$R_1$, $R_2$, $R_3$ und $R_4$ wie oben definiert sind und
Hal ein Halogenatom darstellt, in einem organischen Lösungsmittel bei Temperaturen zwischen 80 und 200°C, gegebenenfalls in Gegenwart katalytischer Mengen einer Mineralsäure, cyclisiert wird
und gewünschtenfalls anschließend eine so erhaltene Verbindung der allgemeinen Formel I, in der $R_4$ wie eingangs definiert ist und $R_1$ ein Wasserstoffatom darstellt, mittels Alkylierung in eine entsprechende Verbindung der allgemeinen Formel I, in der $R_4$ wie eingangs definiert ist und $R_1$ eine Alkylgruppe mit 1 bis 6 Kohlenstoffatomen darstellt, übergeführt wird
oder eine Verbindung der allgemeinen Formel I, in der $R_1$ eine Alkylgruppe mit 1 bis 6 Kohlenstoffatomen und $R_4$ ein Wasserstoffatom darstellen, mittels Alkylierung in eine entsprechende Verbindung

der allgemeinen Formel I, in der $R_1$ eine Alkylgruppe mit 1 bis 6 Kohlenstoffatomen und $R_4$ eine Alkylgruppe mit 1 bis 4 Kohlenstoffatomen darstellen, übergeführt wird
oder eine Verbindung der allgemeinen Formel I, in der $R_1$ und $R_4$ je ein Wasserstoffatom darstellen, mittels Alkylierung in eine entsprechende Verbindung der allgemeinen Formel I, in der $R_1$ und $R_4$ jeweils eine Alkylgruppe mit 1 bis 4 Kohlenstoffatomen darstellen, übergeführt wird.

2. Verfahren gemäß Anspruch 1 zur Herstellung von 1,4,9,10-Tetrahydro-pyrazolo[4,3-e]pyrido-[3,2-b][1,4]diazepin-10-onen der allgemeinen Formel

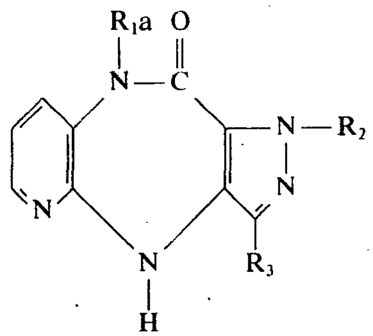

(Ia)

in der
$R_2$ und $R_3$ wie im Anspruch 1 definiert sind, und
$R_1a$ ein Wasserstoffatom oder eine Alkylgruppe mit 1 bis 3 Kohlenstoffatomen bedeutet, dadurch gekennzeichnet, daß ein N-(2-Halogen-3-pyridyl)-4-amino-pyrazol-5-carboxamid der allgemeinen Formel

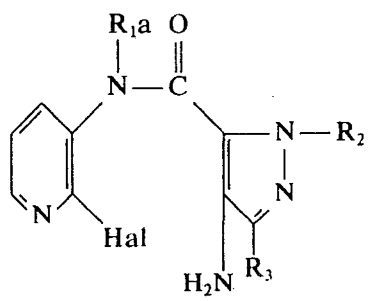

(IIa)

in der
$R_1a$ wie oben sowie Hal, $R_2$ und $R_3$ wie im Anspruch 1 definiert sind, in einem organischen Lösungsmittel bei Temperaturen zwischen 80 und 200°C, gegebenenfalls in Gegenwart katalytischer Mengen einer Mineralsäure, cyclisiert wird und eine so erhaltene Verbindung der allgemeinen Formel I, in der $R_1$ ein Wasserstoffatom ist, gewünschtenfalls anschließend zu einer Verbindung der allgemeinen Formel I, in der $R_1$ eine Alkylgruppe mit 1 bis 3 Kohlenstoffatomen bedeutet, alkyliert wird.

**Claims for the contracting states: BE, CH, DE, FR, IT, LI, LU, NL, SE**

1. 1,4,9,10-Tetrahydro-pyrazolo[3,4-e]pyrido[3,2-b][1,4]diazepin-10-ones of general formula

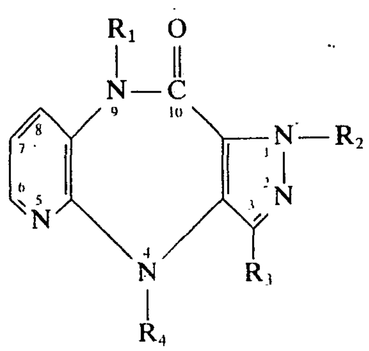

(I)

wherein
$R_1$ represents a hydrogen atom or an alkyl group with 1 to 6 carbon atoms,
$R_2$ represents an alkyl group with 1 to 3 carbon atoms,
$R_3$ represents a hydrogen atom or an alkyl group with 1 to 3 carbon atoms and
$R_4$ represents a hydrogen atom or an alkyl group with 1 to 4 carbon atoms.

2. Compounds of general formula I as claimed in claim 1, wherein
$R_1$ represents a hydrogen atom or an alkyl group with 1 to 3 carbon atoms,
$R_2$ represents an alkyl group with 1 to 3 carbon atoms,
$R_3$ represents a hydrogen atom or an alkyl group with 1 to 3 carbon atoms and
$R_4$ represents a hydrogen atom.

3. Compounds of general formula I as claimed in claim 1, wherein
$R_1$ represents a hydrogen atom, the methyl, ethyl, n-propyl, isopropyl, n-butyl or isobutyl group,
$R_2$ represents the methyl group,
$R_3$ represents a hydrogen atom or the methyl group and
$R_4$ represents a hydrogen atom or the methyl group.

4. A process for the preparation of 1,4,9,10-tetrahydro-pyrazolo[4,3-e]pyrido[3,2-b][1,4]diazepin-10-ones of general formula

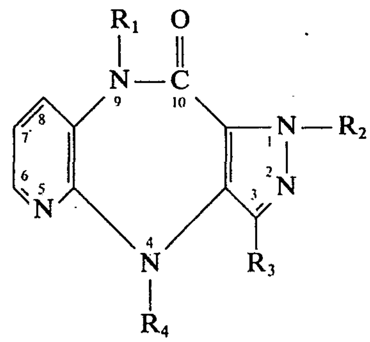

(I)

wherein
$R_1$ represents a hydrogen atom or an alkyl group with 1 to 6 carbon atoms,
$R_2$ represents an alkyl group with 1 to 3 carbon atoms,
$R_3$ represents a hydrogen atom or an alkyl group with 1 to 3 carbon atoms and
$R_4$ represents a hydrogen atom or an alkyl group with 1 to 4 carbon atoms, characterised in that an N-(2-halogen-3-pyridinyl)-4-amino-1H-pyrazole-5-carboxamide of general formula

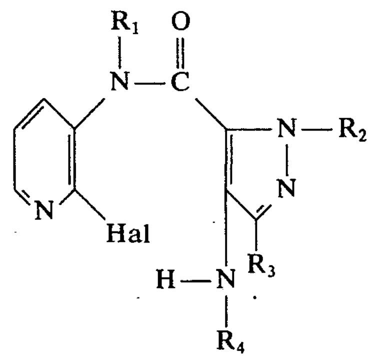

(II)

wherein $R_1$, $R_2$, $R_3$ and $R_4$ are defined as mentioned before and Hal represents a halogen atom, is cyclised in an organic solvent at temperatures between 80 and 200°C, optionally in the presence of catalytic amounts of a mineral acid,
and, if desired subsequently, a compound of general formula I thus obtained, wherein $R_4$ is defined as mentioned before and $R_1$ represents a hydrogen atom, is converted by alkylation into a corresponding compound of general formula I, wherein $R_4$ is defined as mentioned before and $R_1$ represents an alkyl group with 1 to 6 carbon atoms,
or a compound of general formula I,
wherein
$R_1$ represents an alkyl group with 1 to 3 carbon atoms and
$R_4$ represents a hydrogen atom, is converted by alkylation into a corresponding compound of general formula I,
wherein
$R_1$ represents an alkyl group with 1 to 6 carbon atoms and
$R_4$ represents an alkyl group with 1 to 4 carbon atoms,
or a compound of general formula I, wherein $R_1$ and $R_4$ each represent hydrogen atom, is converted by alkylation into a corresponding compound of general formula I, wherein $R_1$ and $R_4$ each represent an alkyl group with 1 to 4 carbon atoms.

5. Pharmaceutical compositions containing one or more compounds of general formula I as claimed in claims 1 to 3 besides the usual carriers and/or excipients.

**Claims for the contracting state: AT**

1. Process for the preparation of 1,4,9,10-tetrahydro-pyrazolo[4,3-e]pyrido[3,2-b][1,4]diazepin-10-ones of general formula

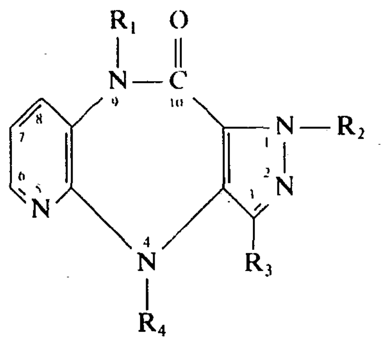

(I)

wherein
$R_1$ represents a hydrogen atom or an alkyl group with 1 to 6 carbon atoms,
$R_2$ represents an alkyl group with 1 to 3 carbon atoms,
$R_3$ represents a hydrogen atom or an alkyl group with 1 to 3 carbon atoms and
$R_4$ represents a hydrogen atom or an alkyl group with 1 to 4 carbon atoms, characterised in that an N-(2-halogen-3-pyridinyl)-4-amino-1H-pyrazole-5-carboxamide of general formula

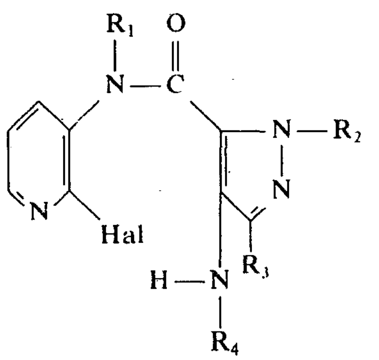

(II)

wherein $R_1$, $R_2$, $R_3$ and $R_4$ are defined as mentioned before and Hal represents a halogen atom, is cyclised in an organic solvent at temperatures between 80 and 200°C, optionally in the presence of catalytic amounts of a mineral acid,
and, if desired subsequently, a compound of general formula I thus obtained, wherein $R_4$ is defined as mentioned before and $R_1$ represents a hydrogen atom, is converted by alkylation into a corresponding compound of general formula I, wherein $R_4$ is defined as mentioned before and $R_1$ represents an alkyl group with 1 to 6 carbon atoms,
or a compound of general formula I,
wherein
$R_1$ represents an alkyl group with 1 to 3 carbon atoms and
$R_4$ represents a hydrogen atom, is converted by alkylation into a corresponding compound of general formula I,
wherein
$R_1$ represents an alkyl group with 1 to 6 carbon atoms and
$R_4$ represents an alkyl group with 1 to 4 carbon atoms,
or a compound of general formula I, wherein $R_1$ and $R_4$ each represent hydrogen atom, is converted by alkylation into a corresponding compound of general formula I, wherein $R_1$ and $R_4$ each represent an alkyl group with 1 to 4 carbon atoms.

2. Process as claimed in claim 1 for preparing 1,4,9,10-tetrahydro-pyrazolo[4,3-e]pyrido[3,2-b]-[1,4]diazepin-10-ones of general formula

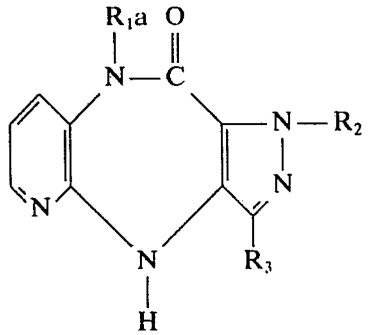

(Ia)

wherein
$R_2$ and $R_3$ are defined as in claim 1 and
$R_1a$ represents a hydrogen atom or an alkyl group with 1 to 3 carbon atoms, characterised in that an N-(2-halo-3-pyridyl)-4-amino-pyrazolo-5-carboxamide of general formula

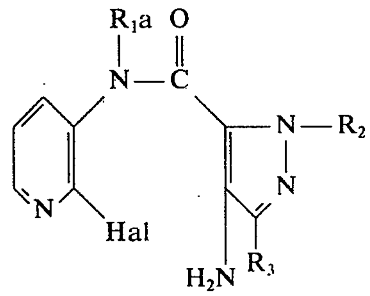

(IIa)

wherein
$R_1a$ is as hereinbefore defined and Hal, $R_2$ and $R_3$ are defined as in claim 1, is cyclised in an organic solvent at temperatures of between 80 and 200°C, optionally in the presence of catalytic quantities of a mineral acid, and a compound of general formula I thus obtained wherein $R_1$ is a hydrogen atom is optionally subsequently converted into a compound of general formula I wherein $R_1$ represents an alkyl group with 1 to 3 carbon atoms.

**Revendications**
Pour les Etats contractants: BE, CH, DE, FR, IT, LI, LU, NL, SE.

1. 1,4,9,10-tétrahydro-pyrazolo[4,3-e]pyrido[3,2-b][1,4]diazépine-10-ones de formule générale:

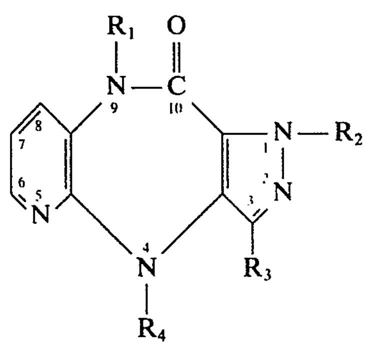

(I)

dans laquelle:
$R_1$ représente un atome d'hydrogène ou un groupe alcoyle avec 1 à 6 atomes de carbone,
$R_2$ représente un groupe alcoyle avec 1 à 3 atomes de carbone,
$R_3$ représente un atome d'hydrogène ou un groupe alcoyle avec 1 à 3 atomes de carbone et
$R_4$ représente un atome d'hydrogène ou un groupe alcoyle avec 1 à 4 atomes de carbone.

2. Composés de formule générale I selon la revendication 1, caractérisés en ce que:
$R_1$ représente un atome d'hydrogène ou un groupe alcoyle avec 1 à 3 atomes de carbone,
$R_2$ représente un groupe alcoyle avec 1 à 3 atomes de carbone,
$R_3$ représente un atome d'hydrogène ou un groupe alcoyle avec 1 à 3 atomes de carbone et
$R_4$ représente un atome d'hydrogène.

3. Composés de formule générale I selon la revendication 1, caractérisés en ce que:
$R_1$ représente un atome d'hydrogène, le groupe méthyle, éthyle, n-propyle, isopropyle, n-butyle ou isobutyle,

$R_2$ représente le groupe méthyle,
$R_3$ représente un atome d'hydrogène ou le groupe méthyle et
$R_4$ représente un atome d'hydrogène ou le groupe méthyle.

4. Procédé pour la préparation de 1,4,9,10-tétrahydro-pyrazolo[4,3-e]pyrido[3,2-b][1,4]diazépine-10-ones de formule générale:

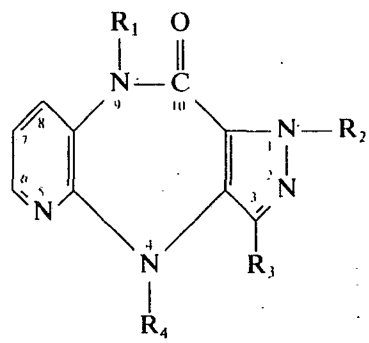

(I)

dans laquelle:
$R_1$ représente un atome d'hydrogène ou un groupe alcoyle avec 1 à 6 atomes de carbone,
$R_2$ représente un groupe alcoyle avec 1 à 3 atomes de carbone,
$R_3$ représente un atome d'hydrogène ou un groupe alcoyle avec 1 à 3 atomes de carbone et
$R_4$ représente un atome d'hydrogène ou un groupe alcoyle avec 1 à 4 atomes de carbone,
caractérise en ce qu'on cyclise un N-(2-halogéno-3-pyridinyl)-4-amino-1H-pyrazol-5-carboxamide de formule générale:

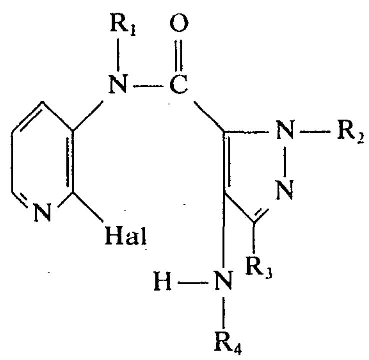

(II).

dans laquelle $R_1$, $R_2$, $R_3$ et $R_4$ sont définis comme plus haut et Hal représente un atome d'halogène, dans un solvant organique à des températures entre 80 et 200°C, éventuellement en présence de quantités catalytiques d'un acide minéral
et si on le désire ensuite, on transforme un composé ainsi obtenu de formule générale I dans laquelle $R_4$ est défini comme au début et $R_1$ représente un atome d'hydrogène, au moyen d'une alcoylation en un composé correspondant de formule générale I dans laquelle $R_4$ est défini comme au début et $R_1$ représente un groupe alcoyle avec 1 à 6 atomes de carbone
ou on transforme un composé de formule générale I dans laquelle $R_1$ représente un groupe alcoyle avec 1 à 6 atomes de carbone et $R_4$ représente un atome d'hydrogène, au moyen d'une alcoylation en un composé correspondant de formule générale I dans laquelle $R_1$ représente un groupe alcoyle avec 1 à 6 atomes de carbone et $R_4$ représente un groupe alcoyle avec 1 à 4 atomes de carbone
ou on transforme un composé de formule générale I dans laquelle $R_1$ et $R_4$ représentent chacun un atom d'hydrogène, au moyen d'une alcoylation en un composé correspondant de formule générale I dans laquelle $R_1$ et $R_4$ représentent chacun un groupe alcoyle avec 1 à 4 atomes de carbone.

5. Médicament contenant un ou plusieurs composés de formule générale I selon les revendications 1 à 3 conjointement à des excipients et/ou adjuvants inertes.

**Revendications**
Pour les Etat contractant: AT.

1. Procédé de préparation de 1,4,9,10-tétrahydro-pyrazolo[4,3-e]pyrido[3,2-b][1,4]diazépine-10-ones de formule générale:

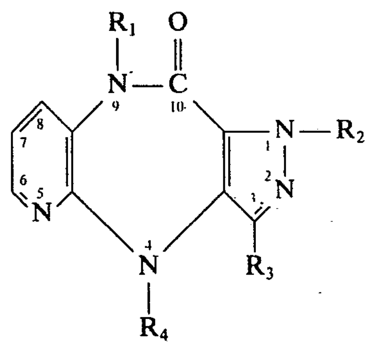

(I)

dans laquelle:
$R_1$ représente un atome d'hydrogène ou un groupe alcoyle avec 1 à 6 atomes de carbone,
$R_2$ représente un groupe alcoyle avec 1 à 3 atomes de carbone,
$R_3$ représente un atome d'hydrogène ou un groupe alcoyle avec 1 à 3 atomes de carbone et
$R_4$ représente un atome d'hydrogène ou un groupe alcoyle avec 1 à 4 atomes de carbone,
caractérisé en ce qu'on cyclise un N-(2-halogéno-3-pyridinyl)-4-amino-1H-pyrazol-5-carboxamide de formule générale:

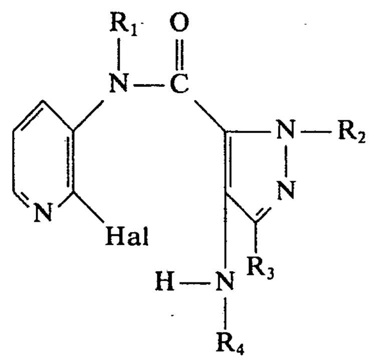

(II)

dans laquelle:
$R_1$, $R_2$, $R_3$ et $R_4$ sont définis comme plus haut et Hal représente un atome d'halogène, dans un solvant organique à des températures entre 80 et 200°C, éventuellement en présence de quantités catalytiques d'un acide minéral
et si on le désire ensuite, on transforme un composé ainsi obtenu de formule générale I dans laquelle $R_4$ est défini comme au début et $R_1$ représente un atome d'hydrogène, au moyen d'une alcoylation en un composé correspondant de formule générale I dans laquelle $R_4$ est défini comme au début et $R_1$ représente un groupe alcoyle avec 1 à 6 atomes de carbone
ou on transforme un composé de formule générale I dans laquelle $R_1$ représente un groupe alcoyle avec 1 à 6 atomes de carbone et $R_4$ représente un atome d'hydrogène, au moyen d'une alcoylation en un composé correspondant de formule générale I dans laquelle $R_1$ représente un groupe alcoyle avec 1 à 6 atomes de carbone et $R_4$ représente un groupe alcoyle avec 1 à 4 atomes de carbone
ou on transforme un composé de formule générale I dans laquelle $R_1$ et $R_4$ représentent chacun un atom d'hydrogène, au moyen d'une alcoylation en un composé correspondant de formule générale I dans laquelle $R_1$ et $R_4$ représentent chacun un groupe alcoyle avec 1 à 4 atomes de carbone.

2. Procédé selon la revendication 1 pour la préparation de 1,4,9,10-tétrahydro-pyrazolo[4,3-e]-pyrido[3,2-b][1,4]diazépine-10-ones de formule générale:

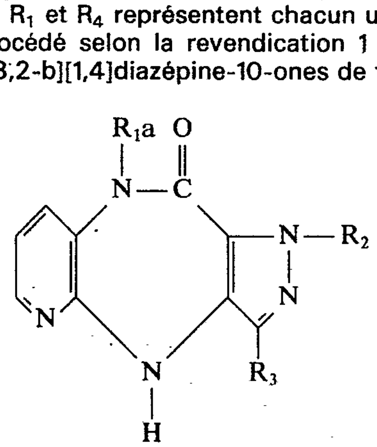

(Ia)

dans laquelle:
$R_2$ et $R_3$ sont définis comme dans la revendication 1 et $R_1a$ représente un atome d'hydrogène ou un

groupe alcoyle avec 1 à 3 atomes de carbone, caractérisé en ce qu'on cyclise un N-(2-halogéno-3-pyridyl)-4-amino-pyrazol-5-carboxamide de formule générale:

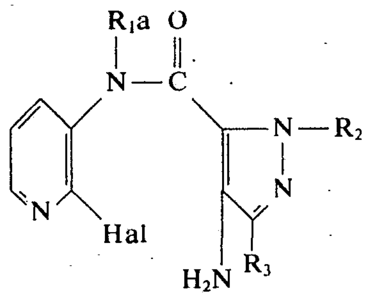

(IIa)

dans laquelle:

$R_1a$ est défini comme plus haut ainsi que Hal, $R_2$ et $R_3$ sont définis comme dans la revendication 1, dans un solvant organique à des températures entre 80 et 200°C, éventuellement en présence de quantités catalytiques d'un acide minéral et en ce qu'on alcoyle un composé ainsi obtenu de formule générale I dans laquelle $R_1$ est un atome d'hydrogène, si on le désire ensuite en un composé de formule générale I dans laquelle $R_1$ représente un groupe alcoyle avec 1 à 3 atomes de carbone.